(19)

Europäisches Patentamt
European Patent Office
Office européen des brevets

(11) **EP 3 541 948 B1**

(12) **EUROPEAN PATENT SPECIFICATION**

(45) Date of publication and mention of the grant of the patent:
**29.04.2020 Bulletin 2020/18**

(21) Application number: **17804140.6**

(22) Date of filing: **16.11.2017**

(51) Int Cl.:
*C12P 7/06* (2006.01)    *C12F 3/10* (2006.01)
*A23K 10/38* (2016.01)    *C12P 7/14* (2006.01)
*A23K 50/30* (2016.01)    *A23K 50/75* (2016.01)

(86) International application number:
**PCT/EP2017/079453**

(87) International publication number:
**WO 2018/091588 (24.05.2018 Gazette 2018/21)**

(54) **METHOD TO IMPROVE THE NUTRITIONAL QUALITY OF FERMENTATION BY-PRODUCTS**

VERFAHREN ZUR VERBESSERUNG DES NÄHRWERTS VON FERMENTATIONSNEBENPRODUKTEN

PROCÉDÉ POUR AMÉLIORER LA QUALITÉ NUTRITIONNELLE DE SOUS-PRODUITS DE FERMENTATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.11.2016 US 201662423559 P**

(43) Date of publication of application:
**25.09.2019 Bulletin 2019/39**

(73) Proprietors:
• **Direvo Industrial Biotechnology GmbH**
  **50829 Köln (DE)**
• **BASF Enzymes, LLC**
  **San Diego, CA 92121 (US)**

(72) Inventors:
• **KRÄMER, Marco**
  **50259 Pulheim (DE)**
• **SCHMITZ, Alexandra**
  **41542 Dormagen (DE)**

(74) Representative: **Roth, Andy Stefan**
**Dr. Roth Patentanwaltskanzlei**
**Kaistrasse 5**
**40221 Düsseldorf (DE)**

(56) References cited:
**WO-A1-2012/084225    WO-A1-2014/127851**
**WO-A1-2014/127852    WO-A1-2014/184054**
**WO-A1-2014/187668**

• **LUANGTHONGKAM P ET AL: "Addition of cellulolytic enzymes and phytase for improving ethanol fermentation performance and oil recovery in corn dry grind process", INDUSTRIAL CROPS AND PRODUCTS, ELSEVIER, NL, vol. 77, 8 October 2015 (2015-10-08), pages 803-808, XP029316122, ISSN: 0926-6690, DOI: 10.1016/J.INDCROP.2015.09.060**

**Description**

**FIELD OF THE INVENTION**

The present disclosure relates to methods for producing ethanol from starch containing material, wherein the in vitro dry-matter digestibility of Dried Distillers Grains with Solubles (DDGS) is improved, wherein the fermentation step is carried out in the presence of a first enzyme composition comprising at least one hemicellulase, wherein the hemicellulase is a xylanase, and wherein the process comprises further the steps of i) subjecting the fermented mash after the fermentation to a second enzyme composition comprising a beta- 1,3 glucanase and a xylanase, ii) separating ethanol by distillation.

**BACKGROUND OF THE INVENTION**

Fermentation products, such as ethanol, are produced by first degrading starch- containing material into fermentable sugars by liquefaction and saccharification and then converting the sugars directly or indirectly into the desired fermentation product using a fermenting organism. Liquid fermentation products such as ethanol are recovered from the fermented mash (often referred to as "beer" or "beer mash"), e.g., by distillation, which separate the desired fermentation product from other liquids and/or solids. The remaining faction, referred to as "whole stillage", is dewatered and separated into a solid and a liquid phase, e.g., by centrifugation. The solid phase is referred to as "wet cake" (or "wet grains" or "WDG") and the liquid phase (supernatant) is referred to as "thin stillage". Dewatered wet cake is dried to provide "Distillers Dried Grains" (DDG) used as nutrient in animal feed. Thin stillage is typically evaporated to provide condensate and syrup (or "thick stillage") or may alternatively be recycled directly to the slurry tank as "backset". Condensate may either be forwarded to a methanator before being discharged or may be recycled to the slurry tank. The syrup consisting mainly of limit dextrins and non-fermentable sugars may be blended into DDG or added to the wet cake before drying to produce DDGS (Distillers Dried Grain with Solubles).

It is known to commercially use the various byproducts and residues derived from the fermentation processes like the ethanol production process. Distillers residues or byproducts, as well as by-products of cereal and other food industry manufacturing, are known to have a certain value as sources of protein and energy for animal feed. Furthermore, the oil from the by-products like DDGS can be recovered as a separate by-product for use in biodiesel production or other biorenewable products are sought.

The by-products like DDG, DDGS or WDG comprises proteins, fibers, fat and unconverted starch. For example DDGS contains typically about 30% of protein. While the protein content is high the amino acid composition is not well suited for monogastric animals if used as animal feed. In general processing of DDGS, especially drying time and temperature are effecting the availability and digestibility of the amino acids, especially lysine.

Furthermore, the by-products are mainly fibrous by-products comprising Crude Fibers (CF), which are structural carbohydrates consisting of cellulose, hemicellulose and indigestible materials like lignin. The structural carbohydrates are not digestible in animal's small intestine. Fibers are characterized and analyzed by different methods and can be divided into crude fibers (CF), neutral detergent fibers (NDF) and acid detergent fibers (ADF). The proportion of cellulose and lignin in the crude fibers fraction also determines the digestibility of crude fibers or its solubility in the intestine. High cellulose and lignin concentrations mean reduced digestibility and vice versa. Hemicelluloses are capable to bind water. The soluble part of fibres the soluble non-starch-polysaccharides (NSP) cannot be digested by monogastric animals like swine and poultry, but increase viscosity, due to their capability to bind water, and are a nutritional constraint, since they can cause moist, sticky droppings and wet litter. The antinutritional effect of soluble NSP's is mainly related to the increase in digesta viscosity. The increased viscosity is slowing down the feed passage rate and hinders the intestinal uptake of nutrients and can lead to decreased feed uptake The viscosity increase a) hinders the intestinal absorption of nutrients and can result in negative effect on the consistency on faces and even symptoms of diarrhea, b) slowing down the feed passage rate and possibly to decreased feed intake. Another effect of NSP's is the so-called "Nutrient Encapsulation". The NSP's in plant cell wall encapsulated starch, protein, oil and other nutrients within the plant cell which is an impermeable barrier preventing full utilization of the nutrients within the cell.

Furthermore the soluble NSP's are responsible for high viscosities during fermentation and are directly influencing separation and drying conditions of fermentation by-products like DDGS in the production process. The bound or encapsulated water in the product is difficult to remove and causes the use of higher drying temperatures and also longer drying time, adversely affecting the quality of temperature-sensitive products like amio acids. The availability and digestibility of essential amino acids in the by-products are lowered by high temperatures and long drying time during production. Examples for NSPs are arabinoxylans, beta-glucans, galactomannans and alpha-galactosides.

As the by-products are used in animal feed for monogastrics animals like pigs and poultry it is important that the by-products have high concentrations of protein with a good amino acid composition and high availability and low soluble fibers content.

Therefore, the two ways for an improvement of a fermentative production plant to increase their efficiency and profitability are an improved production process and the improvement of the quality of the by-products.

In the prior art, a lot of specific processes or treatment methods are described to improve fermentative production processes.

For example, WO 2007/056321 A1 discloses a method of dewatering whole stillage comprising adding enzymes to whole stillage in the ethanol production to improve the solid-liquid separation in the process.

WO 02/38786 describes a process of ethanol production, whereby enzymes are used for thinning the liquefied whole grain mash and the thin stillage. Enzymes are applied to the liquefied mash before the fermentation starts as well as to the thin stillage after centrifugation of the whole stillage.

The US 2006/0275882 A1 describes a process for producing a fermentation product wherein the viscosity of the mash is reduced by the application of enzymes before or during the fermentation. The US 2006/0233864 A1 describes a method for improving the nutritional quality of fibrous by-products for a food manufacturing process, wherein the fibrous by-products like DDGS are inoculated with a filamentous fungus to improve the quality of the by-product.

WO 2012/084225 teaches a process for producing ethanol wherein an enzyme composition comprising 1,3-glucanase and a xylanase is used to improve the in-vitro digestibility of DDGS.

Some ethanol plants use milo, wheat, or barley in the fermentation process, depending on geographical location and time of the year. As a result, nutrient composition can vary among DDGS sources. Because of the near complete fermentation of starch, the remaining amino acids, fat, minerals and vitamins increase approximately three-fold in concentration compared to levels found in corn. Despite the significant increase in crude protein, the poor amino acid balance of DDGS must be addressed when formulating swine and poultry diets.

Therefore, it is an object of the present disclosure to provide improved methods for improving the nutritional quality of the by-products from fermentation processes, namely the in vitro dry-matter digestibility of DDGS.

## SUMMARY OF THE DISCLOSURE

The present disclosure relates to methods for producing ethanol wherein the in vitro dry-matter digestibility of DDGS is improved comprising the steps of:

   i) Converting starch containing material to fermentable sugars,
   ii) Fermentation of the fermentable sugars with a microorganism to fermented mash, wherein the fermentation is carried out in the presence of a first enzyme composition comprising at least one hemicellulase, wherein the hemicellulase is a xylanase,
   iii) Subjecting the fermented mash after the fermentation process to a second enzyme composition comprising a beta- 1,3 glucanase and a xylanase,
   iv) Separation of the ethanol in the fermented mash by distillation.

In a first aspect, the present disclosure relates to methods to improve the in vitro dry-matter digestibility of DDGS of a process for producing ethanol, wherein the process comprises

   i) Converting starch containing material to fermentable sugars,
   ii) Fermentation of the fermentable sugars with a microorganism to fermented mash, wherein the fermentation is carried out in the presence of a first enzyme composition comprising at least one hemicellulase, wherein the hemicellulase is a xylanase,
   iii) Subjecting the fermented mash after the fermentation process to a second enzyme composition comprising a beta- 1,3 glucanase and a xylanase,
   iv) Separation of the ethanol in the fermented mash by distillation.

In a second aspect, the present disclosure also relates methods of of producing ethanol from starch containing material, said method comprising the steps of:

   i) Converting starch containing material to fermentable sugars,
   ii) Fermentation of the fermentable sugars with a microorganism to fermented mash, wherein the fermentation is carried out in the presence of a first enzyme composition comprising at least one hemicellulase, wherein the hemicellulase is a xylanase,
   iii) Subjecting the fermented mash after the fermentation process to a second enzyme composition comprising a beta- 1,3 glucanase and a xylanase,
   iv) Separation of the ethanol in the fermented mash by distillation

The first enzyme composition comprising a xylanase may preferably further comprise a carboxymethylcellulase (CMCase) during the fermentation step and the second enzyme composition used after the fermentation of the fermentable sugars comprises a beta- 1,3 glucanase and a xylanase in order to improve the in vitro dry-matter digestibility of DDGS.

**BRIEF DESCRIPTION OF THE DRAWING**

Figure 1 schematically shows an ethanol production process.

Figure 2 schematically shows an ethanol process including on site fermentation tank for enzyme production based on WDG.

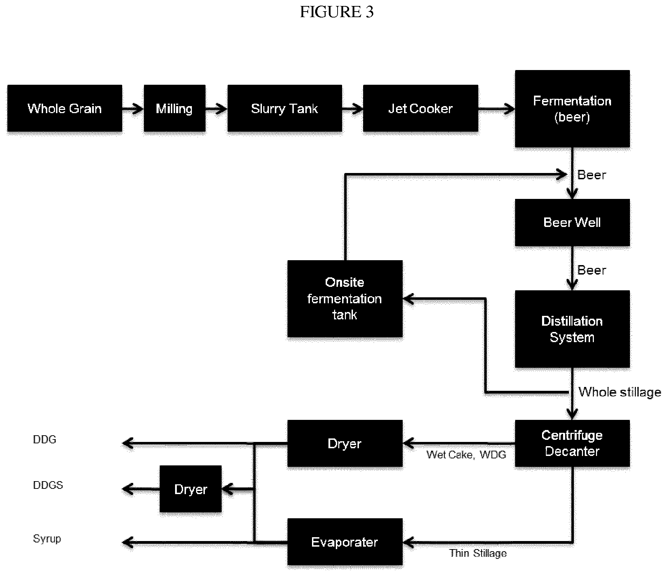

Figure 3 schematically shows an ethanol process including on site fermentation tank for enzyme production based on whole stillage.

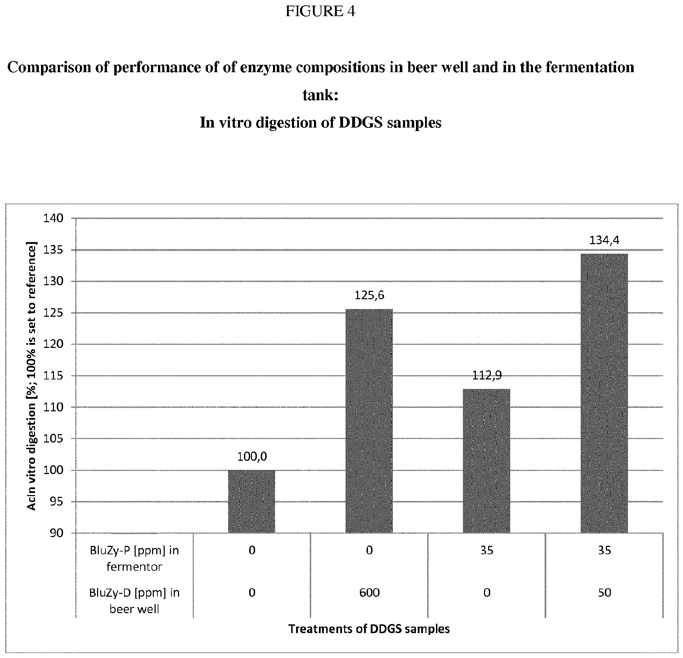

Figure 4 is a diagram showing the *in vitro* digestion of DDGS samples by using the first enzyme composition (Bluzy-P) in the fermentor during the fermentation process and by using the second enzyme composition (Bluzy-D) after the fermentation process in the beer well. Combination of 35 ppm BluZy-P in the fermentation and 50 ppm BluZy-D in the beer well has improved performances compared to the use of 600 ppm BluZy-D alone in the beer well.

**DESCRIPTION OF THE INVENTION**

The object of the present invention is to provide improved fermentative production processes of ethanol and to provide DDGS from the fermentation process with an improved in vitro dry-matter digestibility of DDGS: One aspect of the present disclosure relates to methods for for producing ethanol with improved in vitro dry-matter digestibility of DDGS of, wherein the process comprises converting starch containing material to fermentable sugars, fermentation of the fermentable sugars with a microorganism to fermented mash, wherein the fermentation step is carried out in the presence of a first enzyme composition comprising at least one hemicellulase, wherein the hemicellulase in the first enzyme composition is a xylanase, and wherein the process comprises further the steps of i) subjecting the fermented mash after the fermentation to a second enzyme composition comprising a beta- 1,3 glucanase and a xylanase, ii) separating the desired fermentation product by distillation.

By-products or residues of the fermenting process includes distillers' grain, brewer's grains, dried distiller's grains, dried distiller's solubles, distiller's dried grains with solubles, WDG or/and residues of the cereal processing industry, or mixtures thereof. For example, DDGS are the dried residue remaining after the starch fraction of corn is fermented with selected yeasts and enzymes to produce ethanol and carbon dioxide. After complete fermentation, the alcohol is removed by distillation and the remaining fermentation residues are dried.

Stillage is the product which remains after the mash has been converted to sugar, fermented and distilled into ethanol. Stillage can be separated into two fractions, such as, by centrifugation or screening: (1) wet cake (solid phase) and (2) the thin stillage (supernatant). The solid fraction or distillers' wet grain (DWG) can be pressed to remove excess moisture and then dried to produce distillers' dried grains (DDG). After ethanol has been removed from the liquid fraction, the remaining liquid can be evaporated to concentrate the soluble material into condensed distillers' solubles (DS) or dried and ground to create distillers' dried solubles (DDS). DDS is often mixed with DDG to form distillers' dried grain with solubles (DDGS). DDG, DDGS, and DWG are collectively referred to as distillers' grain(s).

In the present disclosure the first enzyme composition is added during and the second enzyme composition is added after the fermentation in the production process but before the separation by distillation, where the desired fermentation main product is separated from the rest of the fermented mash. The addition of the two enzyme compositions at different time points results in a process for producing ethanol with an improved in vitro dry-matter digestibility of DDGS. This is a result of the combination of the treatment with an enzyme composition comprising xylanase during the fermentation process from starch-containing material using the fermenting microorganism wild-type yeast (*Saccharomyces cerevisiae*) and subsequent treatment with an enzyme composition comprising xylanase and beta 1,3-glucanase in the fermented mash simulating the beer well tank is described. These shows, that the combination of the treatment with an enzyme composition comprising xylanase and preferably with a CMCase during the fermentation process from starch-containing material using the fermenting microorganism wild-type yeast *(Saccharomyces cerevisiae)* and the treatment with an enzyme composition comprising xylanase and glucanase in the fermented mash is more effective and therefore more economic compared to the single treatment with the enzyme composition comprising xylanase and beta 1,3-glucanase in the fermented mash.

The quality of by-products from a fermentative production process like DDG, DDGS or WDG can be improved with the methods according to the present disclosure by increasing the in vitro dry-matter digestibility. For DDGS and other feedstuff increased *in vitro* dry-matter digestibility reflects improved nutritional value (Van Der Klis, J.D., Kwakernaak, C. Proving a concept: An in vitro approach. Journal of Applied (2014) Poultry Research, 23(2), pp 301-305; Weurding, R.E., Veldman, A., Veen, W.A.G., Van Der Aar, P.J., Verstegen, M.W.A. In vitro starch digestion correlates well with rate and extent of starch digestion in broiler chickens (2001) Journal of Nutrition, 131 (9), pp. 2336-2342.; Gehring, C.K., Bedford, M.R., Cowieson, A.J., Dozier III, W.A. Effects of corn source on the relationship between in vitro assays and ileal nutrient digestibility (2012) Poultry Science, 91 (8), pp. 1908-1914.; Valdes, E.V., Leeson, S. Measurement of metabolizable energy in poultry feeds by an in vitro system. (1992) Poultry science, 71 (9), pp. 1493-1503., Boisen, S., Fernandez, J.A. Prediction of the total tract digestibility of energy in feedstuffs and pig diets by in vitro analyses (1997) Animal Feed Science and Technology, 68 (3-4), pp. 277-286.)

In one aspect, the present disclosure relates also to methods for improving the in vitro dry-matter digestibility of DDGS, said method comprising the steps of:

i) Converting starch-containing material to fermentable sugars,
ii) Fermentation of the fermentable sugars with fermenting microorganisms to beer mash,
iii) Subjecting the fermentation medium during the fermentation process to a first enzyme composition comprising at least one hemicellulase, wherein the hemicellulase is a xylanase, and after the fermentation process to a second enzyme composition comprising a beta- 1,3 glucanase and a xylanase,
iv) Separation of the ethanol in the beer mash.

In another aspect, methods for producing ethanol with improved nutritional quality of by-products or residues derived from starch-containing material are described, said method comprising the steps of:

(a) Liquefying a starch-containing material with an alpha-amylase; optionally pre-saccharifying the liquefied material before step (b),
(b) Saccharifying the liquefied material,
(c) Fermenting using fermenting microorganisms; wherein a first enzyme composition comprising a hemicellulase wherein the hemicellulase is a xylanase and a cellulase like a CMCase are added during the fermentation step, and a second enzyme composition comprising a beta-1,3 glucanase and a xylanase is added after the fermentation, in particular added in the beer well.

Also described are methods for producing ethanol comprises the additional steps of:

(a) milling whole grains;
(b) liquefying the product of step (a), in the presence of an alpha-amylase;
(c) saccharifying the liquefied material obtained in step (b);
(d) fermenting the saccharified material obtained in step (c) using a microorganism,
(e) distilling of the fermented and saccharified material obtained in step (d) providing two fraction: 1) an alcohol fraction and 2) a Whole Stillage fraction;
(f) separating the Whole Stillage into two fractions: 1) Wet Grain fraction, and 2) Thin Stillage;
(g) optionally the Thin Stillage is evaporated to provide two fractions: 1) Condensate and 2) Syrup,
(h) separating the desired fermentation by-product from the Wet Grain fraction and/or the Thin Stillage,

wherein a first enzyme composition comprising a hemicellulase wherein the hemicellulase is a xylanase and preferably further comprising a cellulase like a CMCase are added during the fermentation step, and a second enzyme composition comprising a beta- 1,3 glucanase and a xylanase is added after the fermentation, in particular added in the beer well.

**Fermenting microorganism**

The fermenting microorganism may be a fungal organism, such as yeast, or bacteria. Suitable bacteria may, e.g., be *Zymomonas* species, such as *Zymomonas mobilis* and *Escherichia coli.* Examples of filamentous fungi include strains of *Penicillium* species. Preferred organisms for ethanol production are yeasts, e.g., *Pichia* or *Saccharomyces.* Preferred yeasts according to the disclosure are *Saccharomyces* species, in particular *Saccharomyces cerevisiae* or baker's yeast.

Preferred organisms for ethanol production are yeasts, such as e. g. Pichia or Saccharomyces. Preferred yeast according to the disclosure is Saccharomyces species, in particular *Saccharomyces cerevisiae* or baker's yeast. The yeast cells may be added in amounts of $10^5$ to $10^{12}$, preferably from $10^7$ to $10^{10}$, especially $5 \times 10^7$ viable yeast count per ml of fermentation broth. During the ethanol producing phase the yeast cell count should preferably be in the range

from $10^7$ to $10^{10}$, especially around $2 \times 10^8$. Further guidance in respect of using yeast for fermentation can be found in, e. g.,"The alcohol Textbook" (Editors K. Jacques, T. P. Lyons and D. R. Kelsall, Nottingham University Press, United Kingdom 1999), which is hereby incorporated by reference

**Feedstock preparation**

The feedstock for producing the fermentation product may be any starch/hemicellulose-containing material, preferably starch-containing plant material, including tubers, roots, whole grain or any combination thereof. The starch/hemicellulose-containing material may be obtained from cereals. Suitable starch/hemicellulose-containing material includes corn (maize), wheat, barley, cassava, sorghum, rye, potato, or any combination thereof. Corn is the preferred feedstock, especially when the fermentation product is ethanol. The starch-containing material may also consist of or comprise, e.g., a side stream from starch processing, e.g., C6 carbohydrate containing process streams that may not be suited for production of syrups. In particular, the starch-containing material comprises hemicelluloses like lignocellulose. A hemicellulose (also known as polyose) is any of several heteropolymers (matrix polysaccharides), such as arabinoxylans, present along with cellulose in almost all plant cell walls (see Scheller HV, Ulvskov P., Hemicelluloses. // Annu Rev Plant Biol. 2010;61:263-89. doi: 10.1146/annurev-arplant-042809-112315). Hemicelluloses include xylan, glucuronoxylan, arabinoxylan, glucomannan, and xyloglucan. These polysaccharides contain many different sugar monomers. In contrast, cellulose contains only anhydrous glucose. For instance, besides glucose, sugar monomers in hemicellulose can include xylose, mannose, galactose, rhamnose, and arabinose. Hemicelluloses contain most of the D-pentose sugars, and occasionally small amounts of L-sugars as well. Xylose is in most cases the sugar monomer present in the largest amount, although in softwoods mannose can be the most abundant sugar.

Processes for producing fermentation products, such as ethanol, from a starch- or hemicellulose like lignocelluloses-containing material are well known in the art. In a preferred embodiment, the starch/lignocellulose containing material is milled cereals, preferably barley or corn, and the methods comprise a step of milling the cereals before step (a). The preparation of the starch/hemicellulose-containing material such as corn for utilization in such fermentation processes typically begins with grinding the corn in a dry-grind or wet-milling process. Grinding is also understood as milling, as is any process suitable for opening the individual grains and exposing the endosperm for further processing. Wet-milling processes involve fractionating the corn into different components where only the starch fraction enters into the fermentation process. Dry-grind processes involve grinding the whole corn kernels into meal, e.g., by hammer or roller mills, and subsequently mixing the meal with water and enzymes.

Generally two different kinds of dry-grind processes are used. The most commonly used process, often referred to as a "conventional process," includes grinding the starch/hemicellulose-containing material and then liquefying gelatinized starch at a high temperature using typically a bacterial alpha-amylase, followed by "simultaneous saccharification and fermentation" (SSF) carried out in the presence of a glucoamylase and a fermentation organism. Another well-known process, often referred to as a "raw starch hydrolysis" (RSH) process, includes grinding the starch-containing material and then simultaneously saccharifying and fermenting granular starch below the initial gelatinization temperature typically in the presence of an acid fungal alpha-amylase and a glucoamylase.

The term "alpha-amylase" means an alpha-1,4- glucan-4-glucanohydrolase (E.C. 3.2.1.1) that catalyzes the hydrolysis of starch and other linear and branched 1,4-glucosidic oligo- and polysaccharides.

**Preparation of slurry**

The mash may be provided by forming a slurry comprising the milled starch/hemicellulose-containing material and brewing water. The brewing water may be heated to a suitable temperature prior to being combined with the milled starch-containing material in order to achieve a mash temperature of 45-70°C, preferably of 53-66°C, more preferably of 55-60°C. The mash is typically formed in a tank known as the slurry tank.

The aqueous slurry may contain from 10-55 wt-% dry solids, preferably 25-45 wt-% dry solids, more preferably 30-40 wt-% dry solids of the starch-containing material. The slurry is heated to above the gelatinization temperature and an alpha-amylase, preferably a bacterial and/or acid fungal alpha-amylase, may be added to initiate liquefaction (thinning). The slurry may be jet-cooked to further gelatinize the slurry before being subjected to an alpha-amylase in step (a).

**Liquefaction step**

In the liquefaction step the gelatinized starch (downstream mash) is broken down (hydrolyzed) into maltodextrins (dextrins). To achieve starch hydrolysis a suitable enzyme, preferably an alpha-amylase, is added. Liquefaction may be carried out as a three-step hot slurry process. The slurry is heated to between 60-95°C, preferably 80-85°C, and an alpha-amylase may be added to initiate liquefaction (thinning). Then the slurry may be jet-cooked at a temperature between 95-140°C, preferably 105-125°C, for about 1-15 minutes, preferably for about 3-10 minutes, especially around about 5 minutes. The slurry is cooled to 60-95°C and more alpha-amylase may be added to complete the hydrolysis (secondary liquefaction). The liquefaction process is usually carried out at a pH of 4.0 to 6.5, in particular at a pH of 4.5 to 6.

**Saccharification step**

The saccharification step and the fermentation step may be performed as separate process steps or as a simultaneous saccharification and fermentation (SSF) step. The saccharification is carried out in the presence of a saccharifying enzyme, e.g., a glucoamylase, a beta-amylase or maltogenic amylase. Optionally a phytase and/or a protease is added.

Saccharification may be carried out using conditions well known in the art with a saccharifying enzyme, e.g., beta-amylase, glucoamylase or maltogenic amylase, and optionally a debranching enzyme, such as an isoamylase or a pullulanase. For instance, a full saccharification process may last up to from about 24 to about 72 hours, however, it is common to do a pre-saccharification for typically 40-90 minutes at a temperature between 30-65°C, typically about 60°C, followed by complete saccharification during fermentation in a simultaneous saccharification and fermentation (SSF) process. Saccharification is typically carried out at a temperature from 20-75°C, preferably from 40-70°C, typically around 60°C, and at a pH between 4 and 5, normally at about pH 4.5.

The most widely used process to produce a fermentation product, especially ethanol is the simultaneous saccharification and fermentation (SSF) process, in which there is no holding stage for the saccharification, meaning that a fermenting organism, such as a yeast, and enzyme(s), including the hemicellulase(s) and/or specific endoglucanase(s), may be added together. SSF is typically carried out at a temperature from 25-40°C, such as from 28-35°C, from 30-34°C, preferably around about 32°C.

**Fermentation step**

The phrase "fermentation media" or "fermentation medium" refers to the environment in which fermentation is carried out and comprises the fermentation substrate, that is, the carbohydrate source that is metabolized by the fermenting organism(s). The fermentation medium may comprise other nutrients and growth stimulator(s) for the fermenting organism(s). Nutrient and growth stimulators are widely used in the art of fermentation and include nitrogen sources, such as ammonia; vitamins and minerals, or combinations thereof.

In an embodiment, fermentation is ongoing for 6 to 120 hours, in particular 24 to 96 hours.

**Product and By-Product recovery**

According to the invention the fermentation product is ethanol.

The ethanol can be recovered from the fermentation medium using any method known in the art including, but not limited to, chromatography, electrophoretic procedures, differential solubility, distillation, or extraction. For example, ethanol is separated from the fermented cellulosic material and purified by conventional methods of distillation as mentioned above. Ethanol with a purity of up to about 96 vol.% can be obtained, which can be used as, for example, fuel ethanol, drinking ethanol, i.e., potable neutral spirits, or industrial ethanol.

As mentioned above, the by-product or residue is distiller's dried grains with solubles (DDGS).

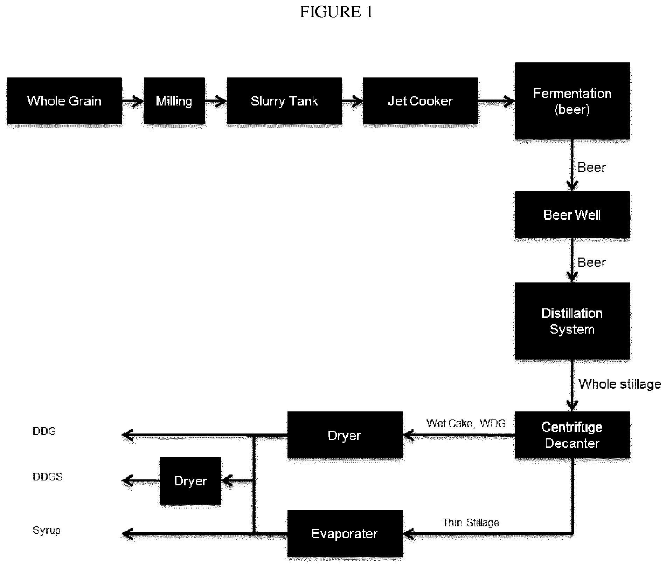

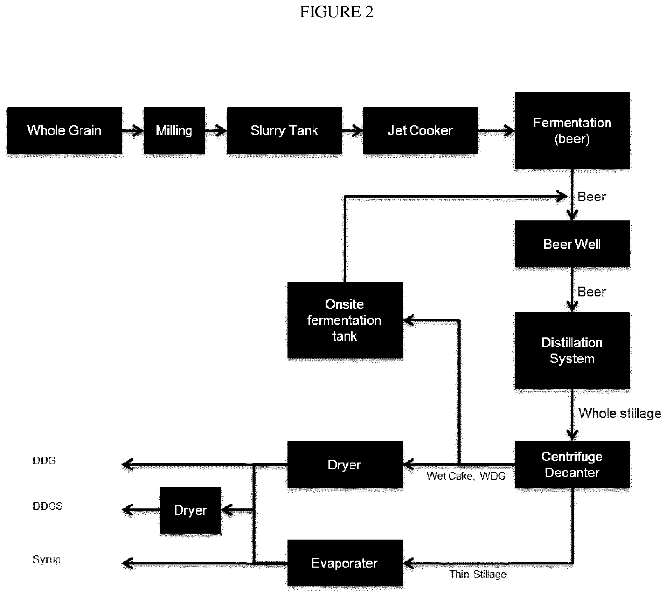

The aqueous by-product (whole stillage) from the distillation process is separated into two fractions, e.g., by centrifugation: wet grain (solid phase), and thin stillage (supernatant). The whole stillage produced by distillation may be separated into wet grain and thin stillage; the thin stillage can be recycled to the starch containing material prior to liquefaction. The thin stillage may be recycled to the milled whole grain slurry. The wet grain fraction may be dried, typically in a drum dryer. The dried product is referred to as distillers dried grains, and can be used as mentioned above as high quality animal feed. The thin stillage fraction may be evaporated providing two fractions (see Fig. 1 and Fig.2), (i) a condensate fraction of 4-6% DS (mainly of starch, proteins, oil and cell wall components), and (ii) a syrup fraction, mainly consisting of limit dextrins and non-fermentable sugars, which may be introduced into a dryer together with the wet grains (from the whole stillage separation step) to provide a product referred to as distillers dried grain with solubles, which also can be used as animal feed. Thin stillage is the term used for the supernatant of the centrifugation of the whole stillage. Typically, the thin stillage contains 4-6% DS (mainly starch and proteins) and has a temperature of about 60-90°C. In another embodiment, the thin stillage is not recycled, but the condensate stream of evaporated thin stillage is recycled to the slurry containing the milled whole grain to be jet cooked.

Further details on how to carry out liquefaction, saccharification, fermentation, distillation, and recovering of ethanol are well known to the skilled person.

Methods for dewatering stillage and for extracting oil from a fermentation product are known in the art. These methods include decanting or otherwise separating the whole stillage into wet cake and thin stillage. See, e.g., U.S. Patent Nos. 6,433,146, 7,601,858, and 7,608,729, and U.S. Application Publication No. 2010/0058649. Furthermore, the thin stillage can be evaporated or condensed into syrup or thick stillage from which the oil can be extracted utilizing centrifugation, filtering, heat, high temperature, increased pressure, or a combination of the same. Another way to extract oil is to lower the pH of the thin stillage or syrup. The use of surfactants to break emulsions also enhances oil extraction. Presses can also be used for dewatering.

Whole stillage typically contains about 10-15 wt-% dry solids. Whole stillage components include fiber, hull, germ, oil and protein components from the starch-containing feedstock as well as non-fermented starch.

The whole stillage is separated into solid and liquid fractions (i.e., wet cake and thin stillage containing about 35 wt-% and 7 wt-% solids, respectively). The thin stillage is often condensed by evaporation into syrup, subsequently recombined with the wet cake and further dried into DDGS for use in animal feed.

Subsequent to fermentation the fermentation product may be separated from the fermentation medium. The beer mash may be distilled to extract the desired fermentation product or the desired fermentation product from the beer mash by micro or membrane filtration techniques. Alternatively the fermentation product may be recovered by stripping.

The ethanol can be optionally recovered from the fermentation medium using any method known in the art including, but not limited to, chromatography, electrophoretic procedures, differential solubility, distillation, or extraction. For example, ethanol is separated from the fermented cellulosic material and purified by conventional methods of distillation as mentioned above. Ethanol with a purity of up to about 96 vol.% can be obtained, which can be used as, for example, fuel ethanol, drinking ethanol, i.e., potable neutral spirits, or industrial ethanol.

## Application of enzyme composition

The saccharification and fermentation steps may be carried out either sequentially or simultaneously. The enzyme composition may be added during saccharification and/or after fermentation when the process is carried out as a sequential saccharification and fermentation process and before or during fermentation when steps (b) and (c) are carried out simultaneously (SSF process).

In the present disclosure the first enzyme composition were added during the fermentation in the production process to the fermentation medium.

The first enzyme composition may be present or added during the optional pre-saccharification step, saccharification step (b), and/or fermentation step (c), or simultaneous saccharification and fermentation. In further examples, the fermentation medium is subjected before, during and/or after the fermentation process to an enzyme composition.

Further, by adding the enzymes according to the present disclosure to the fermentation medium or the beer mash before the distillation step is an advantage since the enzymes in the enzyme compositions are inactivated during the distillation.

## Details on enzyme composition

The first enzyme composition comprises a hemicellulose, wherein the hemicellulase is a xylanase.

In a further embodiment, the first enzyme composition further comprises a cellulase in particular a carboxymethylcellulase (CMCase).

In a further embodiment, the first enzyme composition comprises at least a further enzyme selected from the group consisting of phytase, pectinase, cellulase, glucanase and protease.

The second enzyme composition comprises a beta-1,3 glucanase and a xylanase.

In a further embodiment, the second enzyme composition comprises at least a further enzyme selected from the group consisting of phytase, pectinase, cellulase, glucanase and protease..

In an advantageous embodiment, the first enzyme composition comprises a xylanase and a carboxymethylcellulase (CMCase) and the second enzyme composition comprises a beta- 1,3 glucanase and a xylanase.

## Hemicellulases

Hemicellulases as used herein are enzymes capable to break down hemicellulose like lignocellulose. The hemicellulase used is a xylanase.

Preferred is a hemicellulase which has the ability to hydrolyze hemicellulose under acidic conditions of below pH 7, preferably pH 3-7.

In one aspect, the hemicellulase(s) comprises a commercial hemicellulolytic enzyme preparation. Examples of commercial hemicellulolytic enzyme preparations suitable for use in the present invention include, for example, Cellic® HTec, Cellic® HTec2, Viscozyme®, Ultraflo® and Pulpzyme® HC (from Novozymes A/S), Accellerase® XY, Accellerase® XC (from Genencor Int.), Ecopulp® TX-200A (from AB Enzymes GmbH), Bakezyme® HSP 6000 (from DSM Food Specialties), Depol™ 333P, Depol™ 740L, and Depol™762P (from Biocatalysts Ltd).

In an embodiment of the present disclosure the xylanase may preferably be of microbial origin, such as of fungal origin (e.g., *Aspergillus, Fusarium, Humicola, Meripilus,* and *Trichoderma)* or from a bacterium (e.g., *Bacillus).* In a preferred embodiment the xylanase is derived from a filamentous fungus, preferably derived from a strain of *Aspergillus,* such as *Aspergillus aculeatus;* or a strain of *Humicola,* preferably *Humicola lanuginosa.* Examples of xylanases useful in the methods of the present invention include, but are not limited to *Aspergillus aculeatus* xylanase (GeneSeqP:AAR63790; WO 94/21785), *Aspergillusfumigatusxylanases* (WO 2006/078256), and *Thielavia terrestris* NRRL 8126 xylanases (WO 2009/079210). The xylanase may preferably be an endo-1,4-beta-xylanase, more preferably an endo-1,4-beta-xylanase of GH 10 or GH 11. Examples of commercial xylanases include Shearzyme® 500L, Bio-Feed™ Wheat (from Novozymes A/S), Econase® CE (from AB Enzymes GmbH), Depol™ 676 (from Biocatalysts Ltd.) and Multifect® Xylanase, Spezyme® CP (from Genencor Int.).

Examples of exo-1,4-beta-xylosidase useful in the methods of the present invention include, but are not limited to, *Trichoderma reesei* beta-xylosidase (UniProtKB/TrEMBL accession number Q92458), *Talaromyces emersonii* (SwissProt accession number Q8X212), and *Neurospora crassa* (SwissProt accession number Q7SOW4).

Examples of suitable bacterial xylanases include xylanases derived from a strain of *Bacillus,* such as *Bacillus subtilis,* such as the one disclosed in US patent no. 5,306,633.

Xylanase may be added in an amount effective in the range from $0.16 \times 10^6$ - $460 \times 10^6$ Units per ton beer mash or fermentation medium.

Mannanases as used herein are enzymes capable to break down the part of the hemicelluloses fraction consisting of mannans and heteromannans in plant walls. The mannanases may be any mannanase either endo- and exo-☐-1,4-mannanases but preferably exo-☐-1,4-mannananse, in particular of microbial origin, in particular fungal or bacterial origin.

This may be a mannanase such as a mannanase derived from a strain of a filamentous fungus (e.g., *Aspergillus, Trichoderma, Phanerochaete).* Preferably, the mannanases act on different compositions of lignocellulosic material. Preferred mannanases include endo-acting mannanases, exo-acting mannanases, and combinations thereof. Examples of commercially available mannanases include, for example, Hemicell® (from Elanco Animal Health) and Purabrite® CP (from Genencor Int.).

**Pectinase**

The pectinase may be any pectinase, in particular of microbial origin, in particular of bacterial origin, such as a pectinase derived from a species within the genera *Bacillus, Clostridium, Pseudomonas, Xanthomonas* and *Erwinia,* or of fungal origin, such as a pectinase derived from a species within the genera *Trichoderma* or *Aspergillus,* in particular from a strain within the species *Aspergillus niger* and *Aspergillus aculeatus.* Contemplated commercially available pectinases include Pectinex® Ultra-SPL, Pectinex® Ultra Color (from Novozymes A/S), Rohapect® Classic, Rohapect® 10L (from AB Enzymes GmbH).

Pectinase may be added in an amount effective in the range from $1.4 \times 10^9$ - $23500 \times 10^9$ Units per ton beer mash or fermentation medium.

**Protease**

The proteases may be any protease, in particular of microbial origin, in particular of fungal and bacterial origin. Preferred proteases are acidic proteases, i.e., proteases characterized by the ability to hydrolyze proteins under acidic conditions below pH 7. Suitable acid fungal proteases include fungal proteases derived from *Aspergillus, Mucor, Rhizopus, Candida, Coriolus, Endothia, Enthomophtra, Irpex, Penicillium, Sclerotium* and *Torlopsis.* Commercial proteases include GC 106™ and Spezyme® FAN (from Genencor Int.). Suitable microbial proteases, although not acidic proteases, include the commercially available products Alcalase® and Neutrase® (both from Novozymes A/S), EX-Protin (from C. Schliessmann Kellerei-Chemie GmbH & Co. KG) and Ronozyme® ProAct (from DSM Nutritional Products).

**Cellulase**

The cellulase as used in the present disclosure may be any cellulase, in particular of microbial origin, in particular fungal or bacterial origin such as a cellulase derived from a strain of a filamentous fungus (e.g., *Aspergillus, Trichoderma, Humicola, Fusarium).* Preferably, the cellulase acts on both cellulosic and lignocellulosic material. Preferred cellulases for use in the present invention include endo-acting cellulases, exo-acting celluases and cellobiases, and combinations thereof. Examples of commercially available cellulases suitable according to the present invention include, for example, Celluclast® (from Novozymes A/S), LAMINEX® and Spezyme® CP (from Genencor Int.) and Econase® CE (from AB Enzymes GmbH), Rohalase® BX (from AB Enzymes GmbH), Cellulase 13P (from Biocatalysts Ltd.).

In advantageous embodiments, the first enzyme composition comprises an endoglucanase (1,4-β-D-glucan-4-glucanohydrolase, carboxymethylcellulase or CMCase; EC 3.2.1.4). CMCase are e.g. described in Kotchoni, O.S., W.E. Gachomo, B. Omafuvbe and O.O. Shonukan, 2006. Purification and biochemical characterization of carboxymethyl cellulase (CMCase) from a catabolite repression insensitive mutant of Bacillus pumilus. Int. J. Agric. Biol., 8: 286-292.

Determination of Carboxymethyl-cellulase (CMCase) (Endoglucanase) activity: CMCase activity may be assayed using a modified method described by Wood and Bhat (1998) with some modifications. The CMCase activity was measured by mixing 0.1 mL of enzyme solution with 0.1 mL of 1.0% (w/v) CMC in 10 mM sodium phosphate buffer, pH 7.0 at 37°C for 60 min. The reaction was stopped by adding 1.0 mL 3,5-dinitro salicylic acid (DNS) regent. The mixture was boiled for 10 min cooled in ice and its optical density at 546 nm was determined. The CMCase activity was measure by using a calibration curve for glucose. One unit of CMCase was defined as the amount of enzyme that released 1 $\mu$mol of glucose per min.

Cellulases may be added in amounts effective in the range or from $0.03 \times 10^6$ -$16 \times 10^6$ Units per ton beer or fermentation medium.

**Glucanase**

Glucan and chitin are far more resistant to microbial degradation than cellulose, which is the major constituent of the cell wall of many yeasts and fungi-like organisms. Glucan is predominantly beta-1,3-linked with some branching via 1,6-linkage (Manners et al., Biotechnol. Bioeng, 38, p. 977, 1973), and is known to be degradable by certain beta-1,3-glucanase systems. Beta-1,3-glucanase includes the group of endo-beta-1,3-glucanases also called laminarinases (E.C. 3.2.1.39 and E.C. 3.2.1.6, Enzyme Nomenclature, Academic Press, Inc. 1992). Beta-1,6-glucanases are enzymes hydrolyzing the beta-1,6-linkage in glucan.

The beta-1,3-glucanases as used in the present disclosure may be any 1,3-glucanase, in particular of microbial origin, in particular fungal or bacterial origin such as a beta-1,3-glucanase derivable from a strain of a filamentous fungus (e.g., *Aspergillus, Trichoderma, Penicillium, Humicola*). The beta-1,3-glucanases may preferably be an endo-1,3-beta-glucanase. The beta-1,6-glucanases as used in the present disclosure may be any 1,6-glucanase, in particular of microbial origin, in particular fungal or bacterial origin such as a beta-1,6-glucanase derivable from a strain of a filamentous fungus (e.g., *Aspergillus, Trichoderma, Penicillium, Humicola*). The beta-1,6-glucanases may preferably be an endo-1,6-beta-glucanase.

Examples for commercial available beta-1,3-glucanases suitable according to the present invention include, for example, Rohalase® BX (from AB Enzymes GmbH), Dyadic® Beta Glucanase BP CONC (from Dyadic International (USA), Inc.) and Rapidase® Glucalees (from DSM Food Specialties). Examples for commercial available beta-1,6-glucanases suitable according to the present invention include, for example, ThermoActive™ Pustulanase Cel136 (from Prokazyme Ltd.).

1,3-glucanases and 1,6-glucanases may be added in an amount effective in the range from $0.08 \times 10^6$ - $920 \times 10^6$ Units per ton beer mash or fermentation medium.

**Phytase**

Phytases are enzymes that degrade phytates and/or phytic acid by specifically hydrolyzing the ester link between inositol and phosphorus. Phytase activity is credited with phosphorus and ion availability in many ingredients. A phytase is capable of liberating at least one inorganic phosphate from an inositol hexaphosphate (e.g., phytic acid). Phytases can be grouped according to their preference for a specific position of the phosphate ester group on the phytate molecule at which hydrolysis is initiated (e.g., 3-phytase (EC 3.1.3.8) or 6-phytase (EC 3.1.3.26)). An example of phytase is myo-inositol-hexakiphosphate-3-phosphohydrolase. Phytases can be obtained from microorganisms such as fungal and bacterial organisms. For example, the phytase may be obtained from filamentous fungi such as Aspergillus (e.g., A. ficuum, A. fumigatus, A. niger, and A. terreus), Cladospirum, Mucor (e.g., Mucor piriformis), Myceliop t ora (e.g., M. t ermop ila), Penicillium (e.g., P. ordei (ATCC No. 22053)), P. piceum (ATCC No. 10519), or P. brevi-compactum (ATCC No. 48944), Talaromyces (e.g., T. thermophilus), Thermomyces (WO 99/49740), and Trichoderma spp. (e.g., T. reesei). In an embodiment, the phytase is derived from Buttiauxiella spp. such as B. agrestis, B. brennerae, B. ferragutiase, B. gaviniae, B. izardii, B. noackiae, and B. warmboldiae. In some embodiments, the phytase is a phytase disclosed in WO 2006/043178.

**EXAMPLES**

In the present example the improved nutritional value of DDGS as result of the combination of the treatment with an enzyme composition comprising xylanase during the fermentation process from starch-containing material using the fermenting microorganism wild-type yeast *(Saccharomyces cerevisiae)* and subsequent treatment with an enzyme composition comprising xylanase and beta 1,3-glucanase in the fermented mash simulating the beer well tank is described. For DDGS and other feedstuff increased *in vitro* dry-matter digestibility reflects improved nutritional value (Van Der Klis, J.D., Kwakernaak, C. Proving a concept: An in vitro approach. Journal of Applied (2014) Poultry Research, 23(2), pp 301-305; Weurding, R.E., Veldman, A., Veen, W.A.G., Van Der Aar, P.J., Verstegen, M.W.A. In vitro starch digestion correlates well with rate and extent of starch digestion in broiler chickens (2001) Journal of Nutrition, 131 (9), pp. 2336-2342.; Gehring, C.K., Bedford, M.R., Cowieson, A.J., Dozier III, W.A. Effects of corn source on the relationship between in vitro assays and ileal nutrient digestibility (2012) Poultry Science, 91 (8), pp. 1908-1914.; Valdes, E.V., Leeson, S. Measurement of metabolizable energy in poultry feeds by an in vitro system. (1992) Poultry science, 71 (9), pp. 1493-1503., Boisen, S., Fernandez, J.A. Prediction of the total tract digestibility of energy in feedstuffs and pig diets by in vitro analyses (1997) Animal Feed Science and Technology, 68 (3-4), pp. 277-286.)

In the *in vitro* digestion system dry matter of DDGS (in weight-%) is digested by different digestive enzymes. The more dry matter is digested by the *in vitro* digestion system the more the nutritional value of DDGS has improved.

Therefore an example of increased *in vitro* dry matter digestibility of DDGS as a result of the combination of the treatment with an enzyme composition comprising xylanase during the fermentation process from starch-containing material using the fermenting microorganism wild-type yeast *(Saccharomyces cerevisiae)* and the subsequent treatment with an enzyme composition comprising xylanase and beta 1,3-glucanase in the fermented mash simulating the beer well tank is described.

**Examples**

In one embodiment, the process of the production of DDGS from corn was performed as follows:

**A) Process for producing fermentation products**

a) Reduction of the particle size of the starch-containing material by milling

- Corn (Nr. 19517, Engelter) was milled to < 2 mm particle size (Coffee grinder G2A HD, BUNN)

b) Forming of a slurry comprising the starch-containing material and water

- Heating of 7.8 L warm tap water (water hardness 3.57 mmol/L) to 30°C in a Biostat C fermentor (Sartorius) and stir at 200 rpm

- Addition of 8 mL α-amylase "VF-Kartoffel" (Schliessmann, Nr. 5049)

- Addition of 4.2 kg milled corn to the heated tap water containing the α-amylase to obtain a 35% solid solution with a final volume of 12 L

c) Liquefying of the starch-containing material (Slurry)

- Increase of temperature to 90°C

- Incubation of the fermentor for 90 min at 90°C and 600 rpm

- Cooling of the slurry to 50°C and stir at 700 rpm

d) Saccharifying of the liquefied material obtained (Mash)

- Dilution of 36 g urea (3 g/L) in 60 mL tap water and addition to the 12 L slurry

- Stirring of the slurry containing 3000 ppm urea in the Biostat C fermentor with 700 rpm for 2 hours for even distribution.

- Dilution of 6 mL glucoamylase "Amylase GA 500" (Schliessmann, Nr. 5042) in 25 mL tap water and subsequent addition to the 12 L slurry

- Stirring of the slurry containing glucoamylase in the Biostat C fermentor with 700 rpm for 5 minutes for even distribution. Final product is designated as mash.

- Use corn mash directly after saccharification to start the fermentation to obtain SSF (simultaneous saccharification and fermentation).

e) Fermentation of the liquefied material obtained (Mash)

- Distribution of the mash into 1000 mL shake flasks in 400 g single portions

- Enzyme stock preparation I: 1.0 g of the xylanase (*Xyl40*) with 994 U/mL and 783 U/mL CMCase (see **Table 1**) were added into a 10 mL graduated cylinder - and filled to 10 mL with tap water.

- The enzyme stock was transferred into a 15 mL tube and then stored at 4°C until use within one hour.

- The following volumes of the enzyme stock preparation were added to the 1000 mL shake flasks containing 400 g of the mash. From each set up duplicates were made (designated A and B).

  Setup#I(Shake flasks #1A and #1B): 0 μl of the enzyme stock preparation resulting in 0 g/t of xylanase

  Setup#2 (Shake flasks #2A and #2B): 0 μl of the enzyme preparation resulting in 0 g/t of xylanase

  Setup#3 (Shake flasks #3A and #3B): 560 μl of the enzyme preparation resulting in 140 g/t of xylanase (*Xyl40*)

  Setup#4 (Shake flasks #4A and #4B): 560 μl of the enzyme preparation resulting inleading to 140 g/t of xylanase (*Xyl40*)

- Yeast propagation: 500 mL autoclaved YNB (yeast nitrogen base) medium (7.6 g/L YNB and 2g/L yeast extract) with 10 g/L glucose (pH 5.7) in a 1 L cultivation flasks, which had been inoculated with 2 mL yeast (Ethanol RED, company Fermentis) from a -80°C cryo stock containing 20% glycerol, were incubated for a minimum of 8 hours (30°C, 150 rpm) leading to the yeast culture.

- Inoculation of each shake flask with 16 mL of the yeast culture.

f) Beer production of the fermented material obtained (fermented mash)

- Cultivations were carried out at 30°C at 150 rpm for 45.75 hours to create fermented mash, i.e. beer.

g) Enzymatic processing of the beer (fermented mash)

- Enzyme stock preparation II: 0.5 g of the beta-1,3-glucanase (*Glu40*) with 1362 U/mL and 0.5 g of the xylanase (*Xyl41*) with 10895 U/mL (see: **Table 1**) were added into a 10 mL graduated cylinder - and filled to 10 mL with tap water.

- The enzyme stock was transferred into a 15 mL tube and then stored at 4°C until use within one hour.

- The following volumes of the enzyme stock preparation were added to the 1000 mL shake flasks containing 400 g of beer (i.e. after 48 hours fermentation time). From each set up duplicates were made (designated A and B).

  Setup#1 (Shake flasks #1A and #1B): 0 μl of the enzyme stock preparation leading to 0 g/t of beta-1,3-glucanase and 0 g/t of xylanase

  Setup#2 (Shake flasks #2A and #2B): 2400 μl of the enzyme preparation leading to 600 g/t of beta-1,3-glucanase (*Glu40*) and 600 g/t of xylanase (*Xyl41*)

  Setup#3 (Shake flasks #3A and #3B): 0 μl of the enzyme preparation leading to 0 g/t of beta-1,3-glucanase and 0 g/t of xylanase

Setup#4 (Shake flasks #4A and #4B): 200 $\mu$l of the enzyme preparation leading to 50 g/t of beta-1,3-glucanase (*Glu40*) and 50 g/t of xylanase (*Xyl41*)

- Cultivation was carried out for additional 16.25 hours to simulate beer well conditions.

h) *Dried Distillers Grains with Solubles (DDGS) production*

- *Production of Dried Distillers Grains with Solubles, DDGS:* After total 62 hours of cultivation the content (about 400mL) of the shake flasks containing beer were poured on metal weighing pans and dried for 96 hours at 60°C.

- Parallel set ups of dried DDGS were pooled: DDGS from shake flasks #1A and #1B, #2A and #2B, #3A and #3B, #4A and #4B.

- DDGS was milled for to 1mm particles in a mill (Pulverisette 14 (Company Fritsch))

- Determination of the % of dry weight in DDGS samples: The initial mass (wet weight) of milled DDGS was determined and then DDGS was dried for 24 hour at 105°C giving the 100 % dry weight value. The division of the initial mass divided by the mass value after drying gives the dry matter content of the samples.

**B) Enzyme product activity determination:**

DNSA solution: For the DNSA solution the following compounds were used:

- Dissolving 5.00 g 3,5-Dinitrosalicylic acid (DNSA) in 300 mL deionized $H_2O$

- Addition of 50 mL NaOH/KOH-solution (4M KOH + 4 M NaOH) drop by drop

- Addition of 150 g K-Na-tartrate tetrahydrate

- Cooling of solution to room temperature

- Addition of deionized $H_2O$ to 500 mL final volume

- Solution to be stored in the dark

*a) Xylanase*

Substrate Xylan from beechwood (Sigma X4252)
Buffer: 100 mM sodium acetate, pH 5.0 containing 20 mM $CaCl_2$ and 0.04 g/L Tween20
Substrate was dissolved in the buffer to a concentration of 1.5% (w/v). 1.5 g of xylan from beechwood low viscosity was suspended in 100 ml of buffer was continuously and mixed well. For dissolving the xylan suspension it is autoclaved (121°C for 20min, cooling slowly) together with a magnetic stir bar. For using the exact xylan concentration the xylan suspension is weighed before and after autoclaving and the volume lost by autoclaving was added with autoclaved (121°C for 20min) deionized water.
For the assay 1.5 mL reaction tubes (Eppendorf) were used. The enzymes were diluted in buffer. 200 $\mu$L of the 1.5% xylan substrate was pre heated for 5 minutes at 40°C in a Thermomixer (Eppendorf). Then a 20 $\mu$L enzyme solution was added. A blank was measured replacing enzyme solution with water. Incubation was carried out exactly for 20 min at 40°C, followed by the addition 100$\mu$L of the DNSA solution stopping the enzyme reaction and vigorously vortexing for 15 seconds. Then the sample is stored in an ice-bath.
144 $\mu$L of the stopped substrate - enzyme mix was pipetted into a well of a 96 well PCR microtiter plate (Greiner). For a xylose standard curve 99$\mu$l of xylose standards and 45$\mu$l DNSA reagent were added and mixed with a pipette.
The 96 well PCR plate, which was covered with sticky aluminum foil, which was sealed tightly, was incubated in a Thermal Cycler (BIO-RAD) for 10 minutes at 99°C and 5min for 4°C with heated lid (temperature is calculated from the cycler). Then the 96 well PCR plate was centrifuged in a plate centrifuge at 3000 g for 5min at room temperature (20 - 25°C). 25$\mu$l of the supernatant with room temperature (20 - 25°C) was transferred into a 96well Nunc plate (transparent, flat) into which 78$\mu$l deionized water had been added and mixed with pipette two times. The adsorption was measured at wavelength 540 nm by a micro titer plate reader (Tecan Infinite M1000).
The activity is calculated as Units per $\mu$L or mg of enzyme product. 1 Unit is defined as the amount of formed

reducing ends in $\mu$mol per minute. The enzyme activities are shown in **Table 1.**

*b) Beta-1,3-Glucanase*

Substrates: Barley beta-glucan (low viscosity) (Megazyme P-BGBL)
Buffer: 100 mM sodium acetate, pH 5.0 containing 20 mM CaCl$_2$ and 0.4 g/L Tween20
Substrate was dissolved in the buffer to a concentration of 1.5% (w/v). 1.5 g of Barley beta-glucan low viscosity was suspended in 100 ml of buffer was continuously and mixed well. For dissolving the Barley beta-glucan suspension it is autoclaved (121°C for 20min, cooling slowly) together with a magnetic stir bar. For using the exact Barley beta-glucan concentration the Barley beta-glucan suspension is weighed before and after autoclaving and the volume lost by autoclaving was added with autoclaved (121°C for 20min) deionized water.
For the assay 1.5 mL reaction tubes (Eppendorf) were used. The enzymes were diluted in buffer. 200 $\mu$L of the 1.5% Barley beta-glucan substrate was pre heated for 5 minutes at 40°C in a Thermomixer. Then a 20 $\mu$L enzyme solution was added. A blank was measured replacing enzyme solution with water. Incubation was carried out exactly for 20 min at 40°C, followed by the addition 100$\mu$L of the DNSA solution stopping the enzyme reaction and vigorously vortexing for 15 second. Then the sample is stored in an ice-bath.
144 $\mu$L of the stopped substrate - enzyme mix was pipetted into a well of a 96 well PCR microtiter plate (Greiner). For a glucose standard curve 99$\mu$l of glucose standards and 45$\mu$l DNSA reagent were added and mixed with a pipette.
The 96 well PCR plate, which was covered with sticky aluminum foil, which was sealed tightly, was incubated in a Thermal Cycler (BIO-RAD) for 10 minutes at 99°C and 5min for 4°C with heated lid (temperature 105°C to prevent condensation). Then the 96 well PCR plate was centrifuged in a plate centrifuge at 3000 g for 5min at room temperature (20 - 25°C). 25$\mu$l of the supernatant with room temperature (20 - 25°C) was transferred into a 96well Nunc plate (transparent, flat) into which 78$\mu$l deionized water had been added and mixed with a pipette two times. The adsorption was measured at wavelength 540 nm by a micro titer plate reader (Tecan Infinite M1000).
The activity is calculated as Units per $\mu$L or mg of enzyme product. 1 Unit is defined as the amount of formed reducing ends in $\mu$mol per minute. The enzyme activities are shown in **Table 1.**

**Table 1: Activities in U/mL of xylanase and beta-1,3-glucanase**

| Type | Activity | *Shortname* |
| --- | --- | --- |
| xylanase | 740 U/mL | *Xyl40* |
| xylanase | 9667 U/mL | *Xyl41* |
| beta 1,3-glucanase | 2483 U/mL | *Glu40* |

**C) Analysis of *in vitro* dry matter digestibility of DDGS**

The determination of the *in vitro* dry matter digestibility of DDGS, which has been dried and ground to 1 mm particles, was performed based on the method published by Boisen and Fernandez (Boisen, S., Fernandez, J.A. Prediction of the total tract digestibility of energy in feedstuffs and pig diets by in vitro analyses (1997) Animal Feed Science and Technology, 68 (3-4), pp. 277-286.)
In this process a defined mass of an initial DDGS sample produced by enzymes added to the fermentor and after 45.75 hours to the fermented mash (beer) is treated with the digestive enzymes pepsin and pancreatin, resulting in the liquefaction of the digestible components of DDGS.
The liquefied digestible components of DDGS are separated by centrifugation from the non-digestible compounds of DDGS. The mass of the non-digestible compounds of the DDGS is determined and after subtracting it from the initial DDGS the mass of the digested compounds of DDGS are calculated.
For evaluation the effects of the combination of the treatment with the enzyme composition comprising xylanase during the fermentation process from starch-containing material using the fermenting microorganism wild-type yeast (*Saccharomyces cerevisiae*) and the treatment with an enzyme composition comprising xylanase and beta 1,3-glucanase in the beer well tank the mass of the digested compounds of DDGS was plotted relative to the reference, i.e. without enzyme treatment during the fermentation process and without enzyme treatment of the fermented mash (beer). The reference is set to 100%.

**Performance**

The analysis of each setup was performed in triplicates.

Pepsin digestion

About 0.6 to 0.7 g of DDGS, which was dried and ground to particle sizes of about 1mm, was added into a 100 mL shake flasks containing a magnetic stirrer. 25 mL of 0.1 M sodium phosphate buffer pH 6.0 was added into each shake flask and stirred for 5 minutes on the magnetic stirrer to homogenize the suspension. 10 ml of 0.2 M HCl was added to the suspension, which was adjusted to obtain pH 2.0 by adding 1 M HCl or 1 M NaOH. To this suspension 1 mL of a pepsin solution containing 0.1 mg/mL pepsin (Sigma, P6887, porcine, 500 U/mg) and 0.5 mL chloramphenicol solution in concentration of 0.5 g chloramphenicol/100 ml EtOH was added. The shake flasks were closed with rubber stoppers and incubated at for 1 hour at 60 rpm at 39°C in a shaker.

**Pancreatin digestion**

After pepsin digestion DDGS was digested by pancreatin. 10 mL 0.2 M sodium phosphate buffer pH 6.8 was added to the shake flasks, followed by adding 5 mL of a 0.6 M NaOH solution and adjustment to obtain pH 6.8 by adding 1 M HCl or 1 M NaOH. To this suspension 1 mL of a centrifuged (4000 g for 30 minutes) pancreatin solution containing 100 mg/mL pancreatin (Sigma, porcine, P1750) was added. The shake flasks were closed with rubber stoppers and incubated at for 2 hours at 60 rpm at 39°C in a shaker.

**Determination of the mass of non-digestible compounds of DDGS**

The mass of paper filters (Whatman No. 1, 125 mm Ø, CatNo. 1001125), which were died at 90°C over night, was determined. Then the content of each of the shake flasks was filtered through a dried and weighed filter. In order to obtain also the residual non-digestible compounds the shake flasks were washed with deionized water (about 100 mL), which was previously heated at 40°C. The obtained suspensions were also additionally poured onto the corresponding filters. Finally the filters were dried over night at 90°C and the cooled in a desiccator before weighing them.

**Calculation of the mass of *non-digestible* compounds of DDGS:**

a)

Absolute weight of dried (100% dry weight) DDGS matter af*ter* dry matter *in vitro* dry matter digestion =

(Filter weight of dried filter with digested DDGS) - (Filter weight of empty dried filter)

b)

% of non-digestible compounds of DDGS (based on 100% dry weight) related to *initial* DDGS (based on 100% dry weight) = 100 * Absolute weight of dried (100% dry weight) DDGS matter af*ter in vitro* dry matter digestion [g] / *initial* DDGS (100% dry weight) [g]

**Calculation of the mass of *digestible* compounds of DDGS:**

c) Definition: Digestible compounds of DDGS in % = 100 - % of non-digestible compounds of DDGS (based on 100% dry weight) related to initial DDGS (based on 100% dry weight)

d) Calculation of the mass of digestible compounds of DDGS normalized to the reference in % (Treatment after start of Fermentation (fermentor): 0 g/t xylanase; Treatment after 45.75 h (simulation of beer well): 0 g/t xylanase plus 0 g/t beta 1,3-glucanase):
100 * digestible compounds of DDGS in % / digestible compounds of DDGS in %; which is designated as *in vitro* dry matter digestibility of DDGS [%; 100% is set to the reference DDGS sample; (Setup #1 (pooled DDGS from shake flasks #1A and #1B))].

*Reference DDGS samples:* DDGS made from fermented mash (beer), into which neither a xylanase (*Xyl40*) (0 g/t) had been added into the fermentation process in the fermentor nor the combination of xylanase (*Xyl41*) and beta 1,3-glucanase (*Glu40*) (0 g/t xylanase plus 0 g/t beta 1,3-glucanase) had been added to the fermented mash (beer) after 45.75 h of the fermentation process:

In the depicted examples this is DDGS created from set up #1, i.e. pooled DDGS from shake flasks #1A and #1B.

The results of analysis of *in vitro* dry matter digestibility of the DDGS sample are presented in **Table 2.**

**Table 2** Results of analysis of *in vitro* dry matter digestibility of the DDGS sample. *In vitro* dry matter digestibility of DDGS was performed in triplicates (n=3).

| | *In vitro* **dry matter digestibility of DDGS [%; 100% is set to the reference; (Setup #1 (pooled DDGS from shake flasks #1A and #1B))** | | | | | | |
|---|---|---|---|---|---|---|---|
| | Setup #1 | | Setup #2 | | Setup #3 | | Setup #4 |
| Treatment after start of fermentation (fermentor) | 0 g/t xylanase (*Xyl40*) | | 0 g/t xylanase (*Xyl40*) | | 140 g/t xylanase (*Xyl40*) | | 140 g/t xylanase (*Xyl40*) |
| Treatment after 45.75 h (beer well) | 0 g/t xylanase (*Xyl41*) plus 0 g/t beta 1,3-glucanase (*Glu40*) | | 600 g/t xylanase (*Xyl41*) plus 600 g/t beta 1,3-glucanase (*Glu40*) | | 0 g/t xylanase (*Xyl41*) plus 0 g/t beta 1,3-glucanase (*Glu40*) | | 50g/txylanase (*Xyl41*) plus 50 g/t beta 1,3-glucanase (*Glu40*) |
| | Average (n=3) | Standard Deviation | Average (n=3) | Standard Deviation | Average (n=3) | Standard Deviation | Average (n=3) | Standard Deviation |
| | 100.0 | 6.3 | 125.6 | 6.1 | 112.9 | 3.8 | 134.4 | 1.3 |

**Table 2** shows that the combination of the treatment with an enzyme composition xylanase (Xyl40) (140 g/t) during the fermentation process from starch-containing material using the fermenting microorganism wild-type yeast *(Saccharomyces cerevisiae)* and the treatment with an enzyme composition comprising xylanase (*Xyl41*) and beta 1,3-glucanase (*Glu40*) (50 g/t of both enzymes) in the fermented mash simulating the beer well tank resulted in a higher *in vitro* dry matter digestibility (134.4%, Setup#4) compared to single enzyme treatment in the fermentor (112.9%, Setup#3) or in the beer well (125.6%, Setup#2).

Even a 12-fold increase of the enzyme amount of xylanase (*Xyl41*) and beta 1,3-glucanase (*Glu40*) (600 g/t of both enzymes) in the fermented mash was not able to compensate the effect on DDGS digestibility (125.6%, Setup#2), which was observed by the synergistic effect of treatment in the fermentor with the enzyme composition comprising xylanase (*Xyl40*) and with the enzyme composition comprising xylanase (*Xyl41*) and beta 1,3-glucanase (*Glu40*) in the fermented mash (134.4%, Setup#5).

These results demonstrate, that the combination of the treatment with an enzyme composition comprising xylanase during the fermentation process from starch-containing material using the fermenting microorganism wild-type yeast (*Saccharomyces cerevisiae*) and the treatment with an enzyme composition comprising xylanase and glucanase in the fermented mash is more effective and therefore more economic compared to the single treatment with the enzyme composition comprising xylanase and beta 1,3-glucanase in the fermented mash.

## Claims

1. A method to improve the *in vitro* dry-matter digestibility of Dried Distillers Grains with Solubles (DDGS) in a process for producing ethanol, wherein the process comprises

   i) Converting starch containing material to fermentable sugars,
   ii) Fermentation of the fermentable sugars with a microorganism to fermented mash, wherein the fermentation is carried out in the presence of a first enzyme composition comprising at least one hemicellulase, wherein the hemicellulase is a xylanase,
   iii) subjecting the fermented mash after the fermentation to a second enzyme composition comprising a beta-1,3 glucanase and a xylanase,

   separating the ethanol by distillation.

2. The method according to claim 1, wherein the first enzyme composition further comprises a cellulase.

3. The method according to claim 2, wherein the cellulase is an endoglucanase.

4. The method according to claim 3, wherein the endoglucanase is a carboxymethylcellulase (CMCase).

5. The method according to any one of claim 1 to 4, wherein the starch-containing material is selected from the group consisting of corn, wheat, barley, triticale, cassava, sorghum, rye, potato, or any combination thereof.

6. A method of producing ethanol from starch containing material, said method comprising the steps of:

   i) Converting starch containing material to fermentable sugars,
   ii) Fermentation of the fermentable sugars with a microorganism to fermented mash, wherein the fermentation is carried out in the presence of a first enzyme composition comprising at least one hemicellulase, wherein the hemicellulase is a xylanase,
   iii) Subjecting the fermented mash after the fermentation process to a second enzyme composition comprising a beta- 1,3 glucanase and a xylanase,
   iv) Separation of the ethanol in the fermented mash by distillation.

7. The method according to claim 6, wherein the starch-containing material is selected from the group consisting of corn, wheat, barley, triticale, cassava, sorghum, rye, potato, or any combination thereof.

8. The method according to claim 6 or 7, wherein the microorganism is a yeast, such as a yeast belonging to *Saccharomyces spp.,* in particular *Saccharomyces cerevisae.*

**Patentansprüche**

1. Verfahren zur Verbesserung der *In-vitro*-Verdaulichkeit von Trockenschlempe (DDGS) in einem Verfahren zur Herstellung von Ethanol, wobei das Verfahren einen Fermentationsschritt umfasst, wobei das Verfahren umfasst

   i) Umwandlung von stärkehaltigem Material in fermentierbaren Zucker,
   ii) Fermentation der fermentierbaren Zucker mit einem Mikroorganismus zu fermentierter Maische, wobei die Fermentation in Gegenwart einer ersten Enzymzusammensetzung durchgeführt wird, die mindestens eine Hemicellulase umfasst, wobei die Hemicellulase eine Xylanase ist,
   iii) Aussetzen der fermentierten Maische nach dem Fermentationsprozess einer zweiten Enzymzusammensetzung, die eine Beta-1,3-Glucanase und eine Xylanase umfasst,
   iv) Abtrennung des Ethanols in der fermentierten Maische durch Destillation.

2. Verfahren nach Anspruch 1, wobei die erste Enzymzusammensetzung ferner eine Cellulase umfasst.

3. Verfahren nach Anspruch 2, wobei die Cellulase eine Endoglucanase ist.

4. Verfahren nach Anspruch 3, wobei die Endoglucanase eine Carboxymethylcellulase (CMCase) ist.

5. Verfahren nach einem der Ansprüche 1 bis 4, wobei das stärkehaltige Material ausgewählt ist aus der Gruppe bestehend aus Mais, Weizen, Gerste, Triticale, Maniok, Sorghum, Roggen, Kartoffel oder einer beliebigen Kombination davon.

6. Verfahren zur Herstellung von Ethanol aus stärkehaltigem Material, wobei das Verfahren die folgenden Schritte umfasst:

   i) Umwandlung von stärkehaltigem Material in fermentierbaren Zucker,
   ii) Fermentation der fermentierbaren Zucker mit einem Mikroorganismus zu fermentierter Maische, wobei die Fermentation in Gegenwart einer ersten Enzymzusammensetzung durchgeführt wird, die mindestens eine Hemicellulase umfasst, wobei die Hemicellulase eine Xylanase ist,
   iii) Aussetzen der fermentierten Maische nach dem Fermentationsprozess einer zweiten Enzymzusammensetzung, die eine Beta-1,3-Glucanase und eine Xylanase umfasst,
   iv) Abtrennung des Ethanols in der fermentierten Maische durch Destillation.

7. Verfahren nach Anspruch 6, wobei das stärkehaltige Material ausgewählt ist aus der Gruppe bestehend aus Mais, Weizen, Gerste, Triticale, Maniok, Sorghum, Roggen, Kartoffel oder einer beliebigen Kombination davon.

8. Verfahren nach Anspruch 6 oder 7, wobei der Mikroorganismus eine Hefe ist, wie eine Hefe, die zu *Saccharomyces spp.* gehört, insbesondere *Saccharomyces cerevisae.*

**Revendications**

1. Procédé pour améliorer la digestibilité *in vitro* de matière sèche de drèches et solubles de distillerie (DDS) dans un processus destiné à la production d'éthanol,
   dans lequel le processus comprend

   i) la conversion de matière contenant de l'amidon en sucres fermentescibles,
   ii) la fermentation des sucres fermentescibles avec un microorganisme en moût fermenté, dans lequel la fermentation est effectuée en présence d'une première composition enzymatique comprenant au moins une hémicellulase, dans lequel l'hémicellulase est une xylanase,
   iii) la soumission du moût fermenté après la fermentation à une seconde composition enzymatique comprenant une bêta-1,3 glucanase et une xylanase,
   iv) la séparation de l'éthanol par distillation.

2. Procédé selon la revendication 1, dans lequel la première composition enzymatique comprend en outre une cellulase.

3. Procédé selon la revendication 2, dans lequel la cellulase est une endoglucanase.

**4.** Procédé selon la revendication 3, dans lequel l'endoglucanase est une carboxyméthylcellulase (CMCase).

**5.** Procédé selon l'une quelconque des revendications 1 à 4, dans lequel la matière contenant de l'amidon est choisie dans le groupe constitué de maïs, de blé, d'orge, de triticale, de manioc, de sorgho, de seigle, de pomme de terre, ou de toute combinaison de ceux-ci.

**6.** Procédé de production d'éthanol à partir de matière contenant de l'amidon, ledit procédé comprenant les étapes de :

i) conversion de matière contenant de l'amidon en sucres fermentescibles,
ii) fermentation des sucres fermentescibles avec un microorganisme en moût fermenté, dans lequel la fermentation est effectuée en présence d'une première composition enzymatique comprenant au moins une hémicellulase, dans lequel l'hémicellulase est une xylanase,
iii) soumission du moût fermenté après le processus de fermentation à une seconde composition enzymatique comprenant une bêta-1,3 glucanase et une xylanase,
iv) séparation de l'éthanol dans le moût fermenté par distillation.

**7.** Procédé selon la revendication 6, dans lequel le matériau contenant de l'amidon est choisi dans le groupe constitué de maïs, de blé, d'orge, de triticale, de manioc, de sorgho, de seigle, de pomme de terre ou de toute combinaison de ceux-ci.

**8.** Procédé selon la revendication 6 ou 7, dans lequel le microorganisme est une levure, telle qu'une levure appartenant à *Saccharomyces spp.,* en particulier *Saccharomyces cerevisiae.*

FIGURE 1

FIGURE 2

FIGURE 3

FIGURE 4

**Comparison of performance of of enzyme compositions in beer well and in the fermentation tank:**

**In vitro digestion of DDGS samples**

| | | | | |
|---|---|---|---|---|
| BluZy-P [ppm] in fermentor | 0 | 0 | 35 | 35 |
| BluZy-D [ppm] in beer well | 0 | 600 | 0 | 50 |

Treatments of DDGS samples

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2007056321 A1
- WO 0238786 A
- US 20060275882 A1
- US 20060233864 A1
- WO 2012084225 A
- US 6433146 B
- US 7601858 B
- US 7608729 B
- US 20100058649
- WO 9421785 A
- WO 2006078256 A
- WO 2009079210 A
- US 5306633 A
- WO 9949740 A
- WO 2006043178 A

### Non-patent literature cited in the description

- **VAN DER KLIS, J.D. ; KWAKERNAAK, C.** Proving a concept: An in vitro approach. *Journal of Applied (2014) Poultry Research,* 2014, vol. 23 (2), 301-305
- **WEURDING, R.E. ; VELDMAN, A. ; VEEN, W.A.G. ; VAN DER AAR, P.J. ; VERSTEGEN, M.W.A.** In vitro starch digestion correlates well with rate and extent of starch digestion in broiler chickens. *Journal of Nutrition,* 2001, vol. 131 (9), 2336-2342
- **GEHRING, C.K. ; BEDFORD, M.R. ; COWIESON, A.J. ; DOZIER III, W.A.** Effects of corn source on the relationship between in vitro assays and ileal nutrient digestibility. *Poultry Science,* 2012, vol. 91 (8), 1908-1914
- **VALDES, E.V. ; LEESON, S.** Measurement of metabolizable energy in poultry feeds by an in vitro system. *Poultry science,* 1992, vol. 71 (9), 1493-1503
- **BOISEN, S. ; FERNANDEZ, J.A.** Prediction of the total tract digestibility of energy in feedstuffs and pig diets by in vitro analyses. *Animal Feed Science and Technology,* 1997, vol. 68 (3-4), 277-286
- The alcohol Textbook. Nottingham University Press, 1999
- **SCHELLER HV ; ULVSKOV P.** *Hemicelluloses. // Annu Rev Plant Biol.,* 2010, vol. 61, 263-89
- **KOTCHONI, O.S. ; W.E. GACHOMO ; B. OMAFUVBE ; O.O. SHONUKAN.** Purification and biochemical characterization of carboxymethyl cellulase (CMCase) from a catabolite repression insensitive mutant of Bacillus pumilus. *Int. J. Agric. Biol.,* 2006, vol. 8, 286-292
- **MANNERS et al.** *Biotechnol. Bioeng,* 1973, vol. 38, 977
- Enzyme Nomenclature. Academic Press, Inc, 1992